# EUROPEAN PATENT SPECIFICATION

(11) **EP 0 336 974 B1**
(45) Date of publication and mention of the grant of the patent: **13.04.1994**
(21) Application number: 88908388.7
(22) Date of filing: 30.09.1988
(51) Int. Cl.: C12M 3/00, C12M 1/36

(54) **CELL PROPAGATION APPARATUS**
ZELLENZUCHTVORRICHTUNG
APPAREIL DE CULTURE DE CELLULES

(30) Priority: 01.10.1987 JP 248730/87
(43) Date of publication of application: 18.10.1989
(73) Proprietor: SUMITOMO ELECTRIC INDUSTRIES, LTD., Osaka-shi, Osaka 541 (JP)
(72) Inventor: YONEMORI, Fumihiko, c/o Osaka Works, Konohana-ku, Osaka-shi, Osaka 544 (JP); SHIBATA, Yutaka, c/o Osaka Works, Konohana-ku, Osaka-shi, Osaka 544 (JP); NISHIMURA, Akira, c/o Osaka Works, Konohana-ku, Osaka-shi, Osaka 544 (JP)
(74) Representative: Füchsle, Klaus, Dipl.-Ing.
(86) International application number: JP8801002
(87) International publication number: WO8902913

(56) References cited:
- EP-A- 0 195 088
- DE-A- 2 633 085
- DE-A- 2 813 389
- DE-A- 3 139 310
- JP-A- 5 724 605
- JP-A- 6 188 872
- JP-A-61 152 273
- JP-A-61 202 684
- JP-B- 5 953 028
- JP-B- 5 953 028
- US-A- 4 440 638
- PATENT ABSTRACTS OF JAPAN; p. 109 C 371#

## Description

The present invention relates to a cell proliferation device which separates one or more groups of living cells to be detected and which proliferates the cell group so as to heighten and activate a specific function of said cells, according to the preamble part of claim 1. A similar device is known from DE 2 633 085.

Cultivation of animal cells in vitro is not only used to obtain bioactive substances such as monoclonal antibody, lymphokine and hormone which the animal cells produce, but also used for study of elucidation of a mechanism of cancer development and for study of DNA cloning, activation and differentiation of the cells. Also, the technique of cell cultivation is used in order that, lymphoid cells proliferated and activated by culturing in the presence of lymphokine,i.e., lymphokine activated killer (referred to as LAK hereinafter) or in the presence of a piece of tumor tissue, i.e., cytotoxic T lymphocyte (referred to as CTL hereinafter) are infused, so that the tumor cells can be killed. (see "The New England Journal Vol. 313, No. 23, pages 1485 to 1492)
As described above, the cultivation of animal cells in vitro covers a wide range of application area.

In the prior art, as a cultivation of animal cells, there is e.g. a culture of LAK cells (group) as to be described below.

Collected peripheral blood is separated into two groups, lymphocyte fraction and other fractions, by using differences of specific gravities with the aid of an agent such as Ficoll-Paque, (density gradient centrifugation).

The lymphocyte fraction is collected by a pipet, which is resuspended on a buffer solution such as HANK'S BBS once more and the lymphocyte is purified by repeating two times of centrifugal separation, fractional collection and resuspension (cell washing step). Subsequently, the purified lymphocyte is resuspended on a culture medium such as RPMI-1640 (containing serum) including lymphokine such as interleukin 2.

The culture medium on which the lymphocyte mentioned above is suspended is poured into a roller bottle to be closed and rotating cultivation, stationary cultivation in the culture bottle or cultivation in a culture reactor using hollow fibers is performed.

The culture medium is appropriately exchanged with new culture medium. Also, the temperature of the culture vessel is maintained at 37°C and the pH value of the culture medium is kept constant by aerating CO₂ suitably.

By the operation mentioned above, the number of the lymphocytes is increased and besides the shapes of the lymphocytes become indefinite so that there appear cells having killer activity.

In the case the activated cells mentioned above are used for therapy, the cell suspended medium is collected from the culture vessel and the operations of centrifugation, collection of cell fraction and resuspension on the buffer solution are performed and the same operations as mentioned above are repeated twice, so that the activated cells are purified. After one more centrifugation, the collected cell fractions are resuspended on physiological saline. Furthermore, in many cases, upon sampling, the number of the cells, the phenotype thereof and the killer activity of the cells are measured.

In the case that the activated lymphocytes obtained as described above are used for therapy, the lymphocyte is infused in the patient.

In the prior art, as described above, the collection of blood, cell culture and recovering of activated lymphocyte, including activation and proliferation of lymphocytes, have been manually performed. The number of the activated lymphocytes necessary for therapy must be more than the number of the tumor cells and, for example, in order that tumor cells of generally 1 µm diameter are killed, it is said that an order of more than 10¹¹ activated lymphocytes are required.

However, the number of the lymphocytes which can be collected from a patient at one time of collection is in an order of 10⁸ and according to a manual cultivation such as a cultivation in a roller bottle, the number of lymphocytes to be obtained becomes only generally twice as much as 10⁸.

In order to obtain a sufficient number of activated lymphocytes for therapy, the blood collections and culture operations must be repeated many times for several months, therefore, the amount of work becomes enormous.

Though the cultivation is externally performed, since there are a lot of manual works, there are many risks of contamination of various bacteria, therefore, the manual culture operation suffers from a lack of safety, and the operations mentioned above are performed by a medical doctor in many cases. Because of this, the number of the patients one doctor can take care of is only one or few so that many patients can not be treated by one doctor.

Moreover, since the culture operation is performed manually, the possibility that the cells in culture operation are contaminated by various bacteria is high and since a medical doctor deals with patient blood, there has been a fear that the doctor himself is infected with pathgenic bacteria. Moreover, the culture conditions of lymphocytes are different according to patients and there are differences of works according to doctors and the specifics of the culture cells are different every time, therefore the lymphocyte cells can not be stably obtained.

Similarly in the case that cultured cells are used for study, the amount of work is large and the operations are dangerous and a stable amount of cells can not be obtained, what is a reason why the progress of the study is impaired. Also, in the manual proliferation, the proliferating condition and the activating condition can not be timely measured. Therefore, the optimum culture condition can not be discovered, so that the cells with high activation can not be easily obtained.

The prior art documents DE 2 813 389 and JP 61-88872 disclose the principal of combining the functions of separation, culture, transfer and monitoring in an automatically controlled system.

The documents JP 61-152273, DE 3 139 310, EP 0 195 088 and JP 61-202684 teach the combination of monitoring growth or growth characteristic with control of the culture.

Finally, US 4 440 638 demonstrates the use of electric charge to differentiate cells in a cell culture.

It is an object underlying the present invention to provide a cell proliferation device according to the preamble part of claim 1 which is capable of automatically proliferating and activating cells.

The solution of this object is achieved by a cell proliferation device comprising:
separator means for separating cells;
culture means having a culture tank for cultivating the cells, transfer means for transferring the cells between said separator means and said culture means; and
controller means for controlling the operations of the separator means and the transfer means,
**characterized** in
that said separator means comprise a centrifugal device capable of centrifuging a solution, a pipetting device capable of absorption and discharge of the solution, a manipulator for manipulating said pipetting device, and a sensor for sensing the position of each of cell differentiations after centrifugation, so that the pipetting operation is controlled on the basis of the sensing result of each of the differentiation positions,
that culture monitoring means are provided for measuring the characteristics of the cells stored in said culture means, and
that said controller means calculate the characteristics of the cells obtained by said culture monitoring means and for controlling the culture condition of the culture tank, and for controlling the operations of said separator means, said transfer means and said culture monitoring means.

The centrifugation of the separator may be a continuous centrifugation.

The separation of the cells may be performed by using a density gradient centrifugation.

The separator may be composed of a membrane for separating the cells, a tool for supporting said membrane and a solution path, wherein the cells are separated by filtration.

The above mentioned membrane, tool for supporting the membrane and solution path may be made capable of reverse washing.

The separator may be a separator which separates the cells using differences of quantity of electro charge on the cells.

The separator may be composed of a chamber having electrodes for applying voltage to the solution and of a solution path.

The culture unit may have such a composition that the culture medium can be poured into the culture tank and discharged from the tank.

The culture tank may be provided detachably to the culture unit and the transferring means may be so constituted as to transfer the culture tank between the culture unit and the separator.

Each of the operations of the separator, culture unit and transferring means may be performed in an contamination-free way. The cell proliferation device makes it possible that a group of necessary cells is separated among a group of entered cells in the separator unit and is cultured in the culture unit, monitoring the culture condition of the cells by the culture monitoring unit, and the change of the culture condition and the timing of recovery of the cells are judged by the controller based on the monitoring result of the cell culture condition, and the administration of the bioactive substance is controlled in the culture unit in response to a signal sent from the controller, and the recovery of the cells is performed in the separator unit in response to a signal sent from the controller.

Moreover, a density gradient centrifugation may be used in the operation of the separator. In addition, a separation method with a membrane may be used in the separator. In addition, an electrophoresis may be used in the separator. Moreover, a continuous culturing method may be used in the culture unit. Furthermore, in the culture monitoring unit, a cytotoxic assays for monitoring the activity of the culture cells may be used. In addition, in the culture monitoring unit, a monitor for monitoring the activity of the culture cells or an image processing method for distinguishing cells in a specific subgroup may be used.

Means for sampling the cell suspension in the culture tank may be furthermore provided.

The culture monitoring unit may comprise means for dyeing the cells or means for labelling the cells.

The culture monitoring unit may comprise measuring means for measuring the characteristics of the cells.

The measuring means may be means for processing the images of the cells formed through a microscope.

The measuring means may comprise a light measuring device.

The measuring means may be means for measuring the amount of radioisotope.

The control of the culture condition may be performed by controlling the amount of the administration of the bioactive substance using the measurement result of the characteristics of the cells.

The control of the culture condition may be performed by controlling the exchange of the culture medium using the measurement result of the characteristics of the cells.

The timing of the recovery of the cells may be controlled using the measurement result of the characteristics of the cells.

The characteristics of the cells to be measured may be the number of the cells or the density of the cells.

The characteristics of the cells to be measured may be the cytotoxicity.

The characteristics of the cells to be measured may be the kind of the cells or the phenotype of the cells.

The characteristics of the cells to be measured may be the shapes of the cells, sizes of the cells or the degree of the deformation of the cells.

At least one of the pH value, temperature, humidity and the CO₂ density of the medium may be measured so that the measured value may be controlled in a feedback manner.

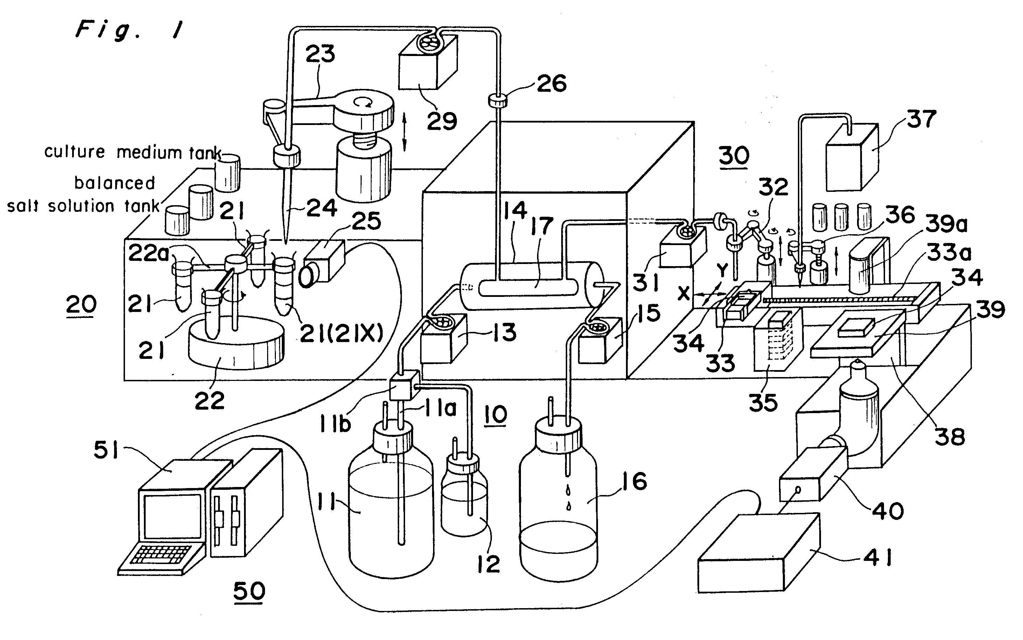

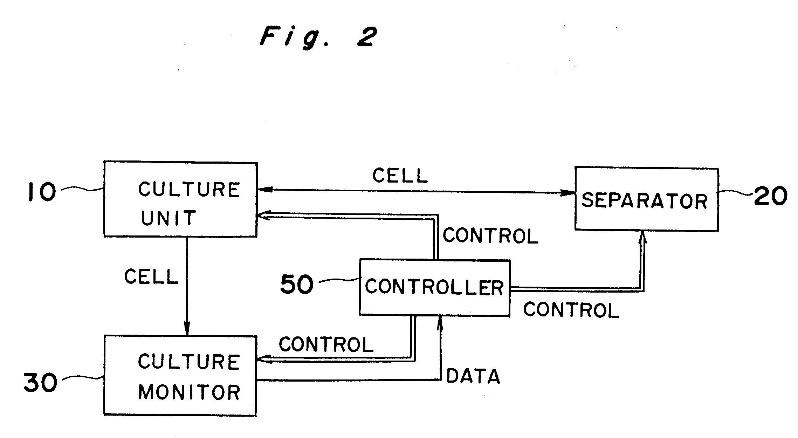

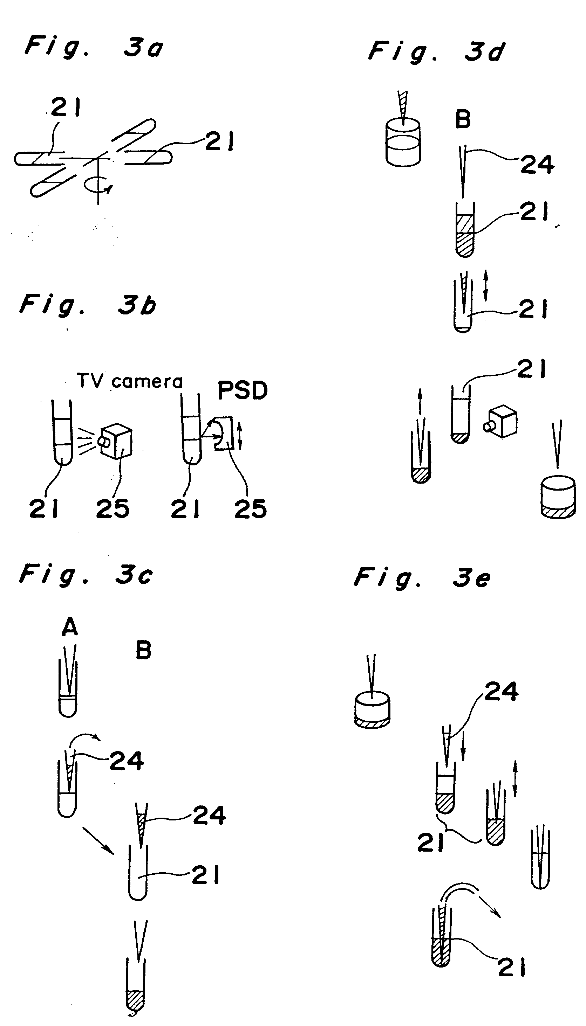

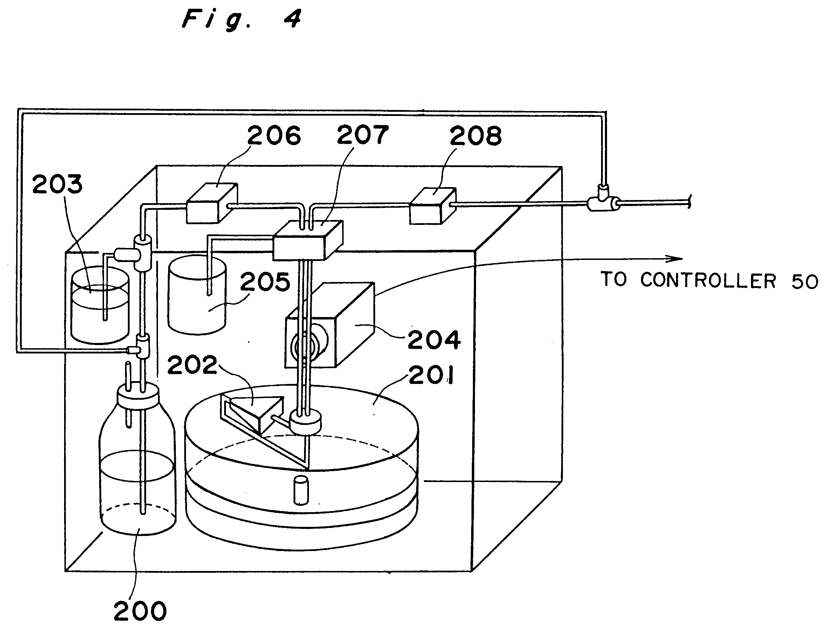

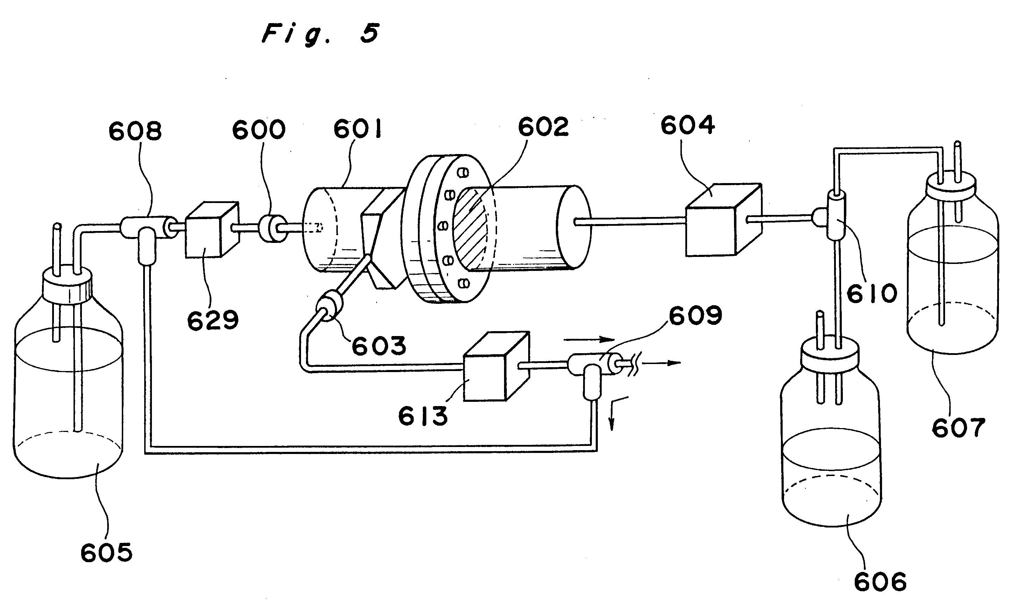

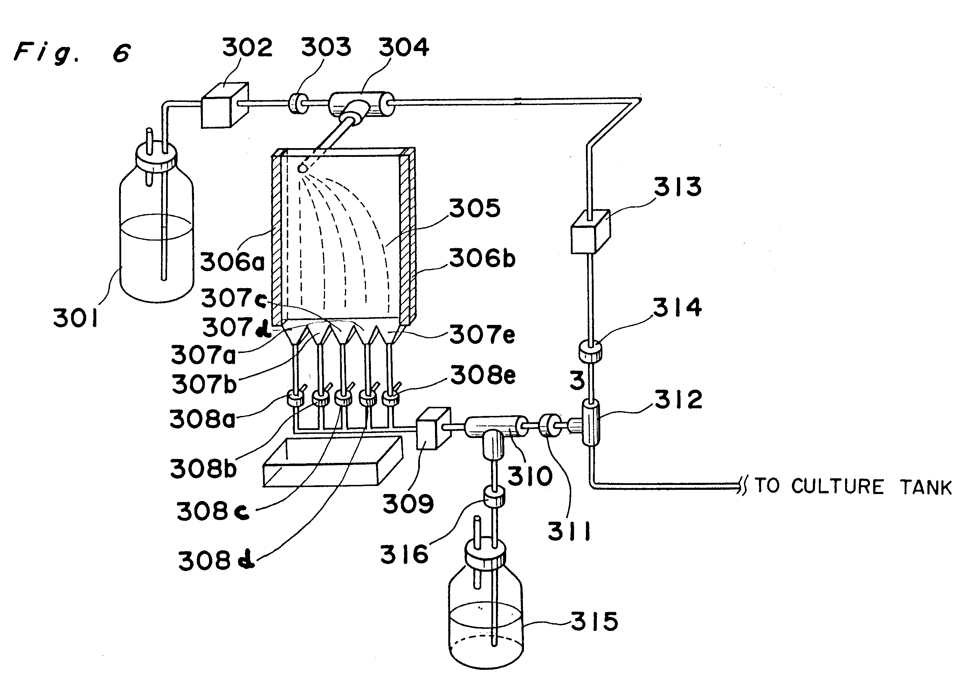

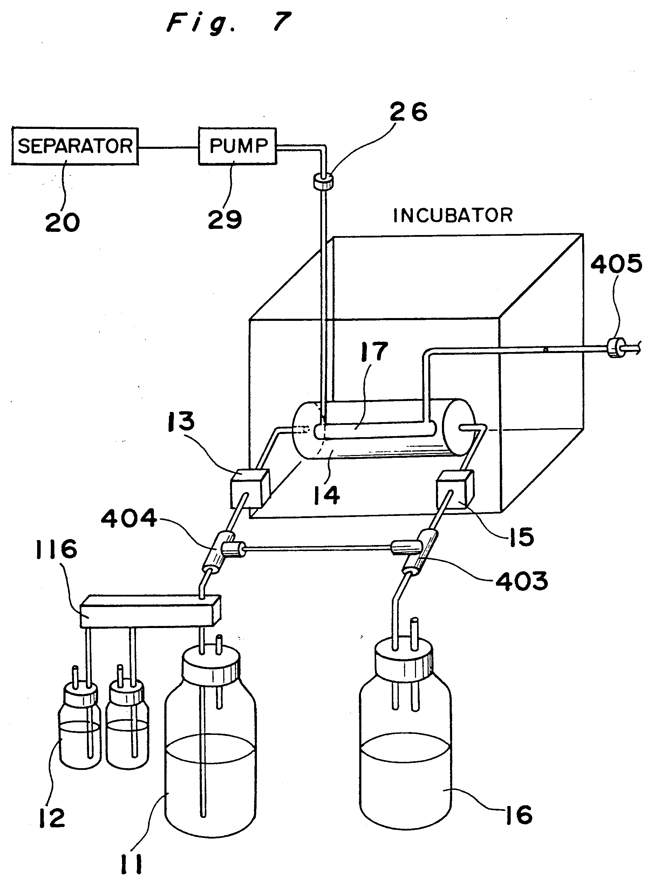

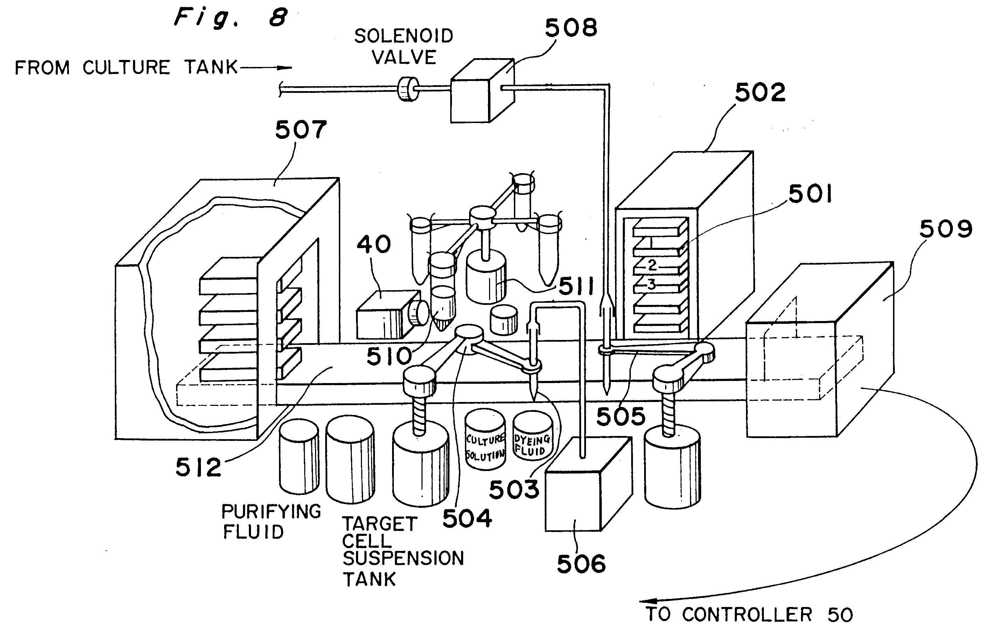

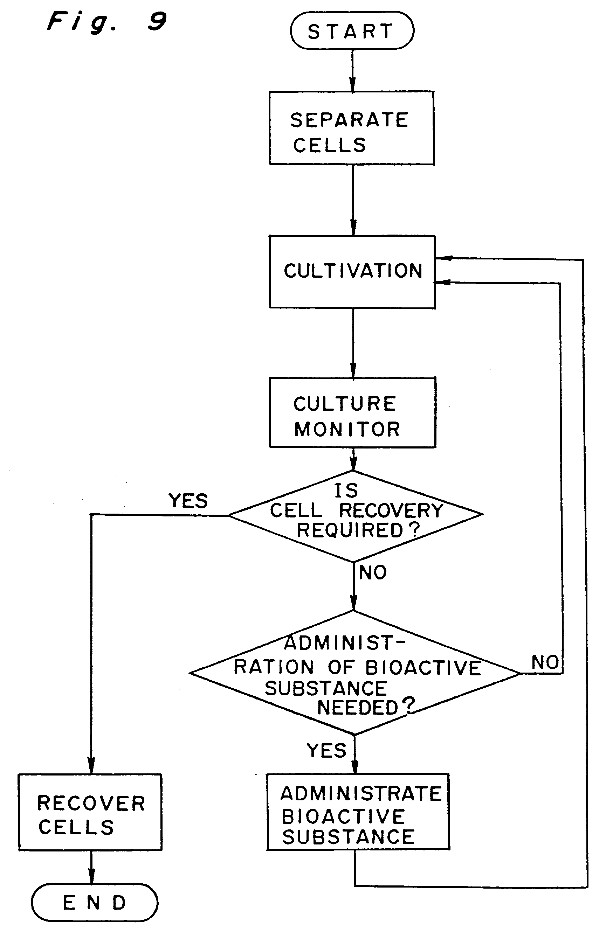

Fig. 1 is a schematic perspective view showing an embodiment of a culture device according to the present invention, Fig. 2 is a block diagram showing the culture device according to the present invention, Figs. 3a to 3e are schematic diagrams showing the essential parts of the operation of the centrifugal unit, Fig. 4 is a perspective view showing another embodiment of the separator unit, Figs. 5 and 6 are perspective views showing further embodiments of the separator unit, Fig. 7 is a schematic view showing another embodiment of the culture unit, Fig. 8 is a schematic view showing another embodiment of the monitoring unit, and Fig. 9 is a flow chart showing an essential part of the operation.

As shown in Figs. 1 and 2, the device according to the present invention comprises a culture unit 10, separator unit 20, a monitoring unit 30 and a controller 50 composed of a microcomputer e.g. for controlling each of the units. In the culture unit 10, predetermined culture medium stored in a culture medium bottle 11 is drawn out through a pipe 11a and is mixed with additive fluid drawn out from an additive fluid bottle 12 in a mixing unit 11b and the mixed solution is transferred to a culture tank 14 by a pump 13. Excessive solution is discharged to a discard tank 16 through a pump 15.

In the separator unit 20, there are provided a plurality of vessels 21 storing various kinds of culture medium, wherein each of the vessels 21 is mounted to a centrifugal device 22. A pipet 24 is mounted on a manipulator 23 so as to be moved up and down, whereby the pipet 24 can be pulled out of or inserted in the vessels 21 so that the desired culture medium is drawn out by a pump 29 and is transferred into a culture pipe 17 provided in the culture tank 14.

In order to monitor the content of a vessel 21X hanged by one of rotation arms 22a of the centrifugal device 22, there is provided a television camera for monitoring or a position sensitive diode (PSD) 25 and an image signal obtained by the television camera or position sensitive diode (PSD) 25 is sent to a monitoring television 51 of the controller 50, so that the content of the vessel 21X can be monitored on the monitoring television 51.

In the monitor unit 30, the culture medium containing cells cultured in the culture pipe 17 is drawn out by a pump 31 and the culture medium is transferred every predetermined amount by a manipulator 32 into a storing vessel 34 disposed on an X-Y table 33 which is movable in X and Y directions.

The storing vessel 34 is transferred to the right in Fig. 1 by moving the X-Y table 33 and is stored in or taken out of a stock room 35 in turn.

There is provided a manipulator 36 above the transfer path of the X-Y table 33 for infusing dyeing fluid, whereby the dyeing fluid can be infused into the culture medium stored in the storing vessel 34 as mentioned above by using a pump 37.

There is provided a microscope 38 at a position shifted in the Y direction from the transfer path 33a for the X-Y table 33, so that an image of the culture medium in the storing vessel 34 can be magnifiably seen by transferring the storing vessel 34 onto a microscope table 39. The image magnified by the microscope 38 is taken by the television camera 40 and the image signal thereof is sent to an image processing device 41 and the image signal obtained by the image processing device 41 is transferred to the monitoring television 51 of the controller 50 so as to be displayed.

As shown in Fig. 1, the separating operation of the culture medium in the case that the centrifugal device 22 is used as the separator unit 20 will be described hereinafter.

### [CENTRIFUGATION]

The culture medium to be separated is infused in each of vessels 21, subsequently, the centrifugal device 22 is driven in response to the command signal of the controller 50 so that the arms 22a are rotated and the vessels 21 are oriented in the horizontal direction due to the rotational centrifugal force (shown in Fig. 3a), whereby the cells are differentiated. Then, the position where required cells are differentiated is sensed. A sense signal and a signal representing the differentiation position are obtained through the television camera or position sensitive diode (PSD) 25 by using the difference of the brightness of each of the differentiated portions, difference of the absorbances or difference of the amount of the reflection (shown in Fig. 3b). The controller 50 drives the manipulator 23 on the basis of the sensed position information and moves the tip end portion of the pipet 24 to the differentiated position of the required cells. Subsequently, the cells are absorbed by the pump 29. Next, the tip of the pipet 24 is moved to another centrifugal vessel position. The sucked cells are discharged by a pump.

Furthermore, balanced salt solution is absorbed from a balanced salt solution tank.

Next, the balanced salt solution is discharged into a centrifugal vessel B so as to be stirred and centrifuged so that a differentiated position of the cells is sensed in a manner similar to the sensing operation mentioned above.

In addition, the supernatant fluid is sucked and discarded. The culture medium in the culture medium tank is sucked and discharged in the centrifugal vessel B. In addition, the culture medium is stirred and the pipet is moved to the bottom portion of the vessel so that the culture medium containing the cells is sucked. (see Figs. 3c, d and e)

### [RECOVERY OPERATION]

A solenoid valve 26 is opened so that the cell suspension in the culture pipe 17 is transferred to the pipet 24.

Subsequently, the centrifugal and differentiation process and cleaning process are performed similar to those in the above mentioned separating process.

Next, the case of using a continuous centrifugal device shown in Fig. 4 as a separator portion is explained.
(A) PREPARATION FOR A CENTRIFUGAL OPERATION: Density gradient centrifugation substance such as Ficoll-Paque is infused in a chamber 202 provided in a rotor 201 from a fluid bottle 203 through a pump 206. The cell suspension in the culture pipe 17 is infused in a fluid tank 203 or the chamber 202.
(B) CENTRIFUGATION: A continuous centrifugal rotor 201 is driven.
(C) SENSE START: The sensing of a group of required cells by a sensor 204 is started.
(D) TRANSFER OF SOLUTION: The differentiated solution in the chamber 202 is transferred by a pump 208.
(E) DIFFERENTIATION: An excessive differentiated solution is transferred to a fluid tank 205 by switching a valve 207 and the desired differentiated solution is transferred to the next process through the pump 208.

Next, an operation of a membrane separation device shown in Fig. 5 as a separator portion 20 is explained.
(A) FLUID PATH SETTING: Three-way valves 608, 609 and 610 are so opened that the fluid flows from a fluid bottle 605 to a discard bottle 606 through a filter tank 601 with a solenoid valve 600 opened, 603 closed in response to a signal sent from the controller 50 in the case of the separating operation and that the fluid flows from the culture tank 14 to the discard tank 606 through the filter tank 601 in the case of the recovery operation. Each of the three-way valves is switched by an electromagnetic solenoid (not shown) in response to the control signal sent from the controller 50.
(B) FILTRATION: The pump 629 and pump 604 are driven by a signal from the controller 50 so that the cell suspension containing the cells flows through the filter 602 from the fluid bottle 605 or from the culture pipe 17. Then, the cells remain in the front stage of the membrane filter 602. The diameter of the mesh of the filter 602 through which grains can pass is profitably selected at any one of the diameters among 1 µm to several 10 µm depending on the kind of cell. As a material of the filter, for example, materials of cellulose group, polycarbonate and fluorine group materials may be used. The filter may be made of such material like nylon wool that the cells can be absorbed or removed depending on the condition of the fluid.
(C) REVERSE CLEANING: The direction of the fluid flow is changed by a signal from the controller 50 so that the fluid flows from a buffer fluid bottle 607 to a solenoid valve 603 through a filter 602. The solenoid valve 600 is closed and valve 603 is opened. The pumps 604 and 613 are driven in response to the signal sent from the controller 50 so that the cells are transferred to the culture pipe 17 (in the case of separation) or to the fluid bottle 605 (in the case of recovery).

Fig. 6 shows the case of separating by using an electrophoresis device.

The cell solution drawn out from a fluid bottle 301 through a pump 302 is transferred to an electrophoresis chamber 305 through a three-way joint 304. The cells are discharged between electrodes 306a and 306b which are provided so as to oppose each other in the electrophoresis chamber 305. There is applied a predetermined D.C. voltage between the electrodes 306a and 306b. The cells are separated in each kind of the cells according to the principle of the electrophoresis and the separated cells fall in any one of separation chambers 307a to 307e. Subsequently, one of solenoid valves 308a to 308e provided below the separation chambers 307a to 307e respectively is selectively opened, whereby the desired cells can be independently taken out.

The taken out cells are transferred to a three-way valve 310 through a pump 309 and transferred to the culture pipe 17 (shown in Fig. 1) in the culture tank 14 through a three-way valve 312 by opening a solenoid valve 311.

Moreover, during the separation, a pump 313 is stopped, a solenoid valve 314 is closed and a solenoid valve 316 to a recovery bottle 315 is closed.

When the cells are recovered from the electrophoresis tank 305, the solenoid valve 311 is closed and the solenoid valve 316 is opened, whereby the cells from the electrophoresis chamber 305 are transferred to the recovery bottle 315.

Next, the operation of the culture portion 10 is described with reference to Fig. 7.
(A) CELL STOCK: The pump 29 (shown in Fig. 1) is driven and a solenoid valve 26 is opened so that the cells to be cultured are transferred from the separator unit 20 to the culture pipe 17 in the culture tank 14 so as to be stocked.
(B) START OF CULTIVATION: The pump 13 is driven and the culture medium is continuously infused in the culture tank 14. The excessive culture medium is discarded in the discard tank 16 by driving the pump 15. After the culture medium is infused in the culture tank through the culture pipe 17, pump 15, three-way valve 403, three-way valve 404, pump 13 and the culture tank 14, the same solution may be circulated in a predetermined period. The surface of the culture pipe 17 is preferably made of semi-permeable membrane (the materials of which may be e.g. cellulose group, polyolefin,polysulfone, polyvinyl and fluorine group materials) or made of mesh materials through which the cells can not be passed but the culture medium and discard materials can be passed.
(C) ADDITION OF BIOACTIVE SUBSTANCE: The additive fluid may be mixed with the culture medium changing the rate of the mixture according to the cell active data. In this case, a plurality of kinds of the additive fluid may be added.
(D) SAMPLING: A solenoid valve 405 is opened at regular intervals and the cell suspension containing cells under the culture operation is sampled from the culture pipe 17 in the culture tank 14 for monitoring.
(E) RECOVERY: According to the cell active data (e.g. density of cells), the cell suspension containing cells in the culture tank 14 is transferred to the separator unit 20.

Next, the operation of the culture monitoring unit 30 is described with reference to Fig. 1.
(A) PREPARATION FOR SAMPLING: The transfer path 33a and the X-Y table 33 are driven in response to the transfer path signal and the X-Y table signals so that the cell suspension is moved to the stock position. The measurement vessel is picked out of the stock room 35 so as to be secured by a fixing zipper in response to a fixing zipper signal. The measurement vessel 34 is moved to the infusion position in response to the transfer path signal and X-Y table signals.
(B) SAMPLING INFUSION: The pump 31 is driven by a infusion manipulator signal and pump signal so that the cell suspension containing cells is transferred from the culture pipet 17 in the culture tank 14 being infused into the measurement vessel 34 disposed in a predetermined position by a pipet manipulator 32.
(C) DYEING: The supernatant fluid of the sampling suspension is excluded by a pump 37 and dyeing manipulator 36. (Infusion, absorption and exclusion of fixative may be included.) The dyeing fluid is infused in the measurement vessel by the pump 37 and the dyeing manipulator 36. The dyeing fluid is absorbed and excluded by the pump 37 and the dyeing manipulator 36. The purification fluid is infused, absorbed and excluded by the pump 37 and dyeing manipulator 36.
(D) PREPARATION FOR MEASUREMENT: The measurement vessel 34 is transferred to the microscope stage 39 in response to the transfer path signal, X-Y table signals, light source signal and image processing device driving signal. A light source 39a is turned on. The image processing device 41 is driven and the image information obtained by the television camera 40 is observed by the monitor 51.
(E) MEASUREMENT: The image processing device 41 is driven in response to the image processing signal and X-Y table light source signal so that e.g. the number of the cells, deformation degree and kinds of the cells are measured by monitoring the display screen of the monitoring television 51 or by the data processing in the controller 50.

In addition, the operations including sampling, preliminary processing and measurement are performed in response to the signal sent from the controller 50 at every regular interval.

Moreover, the number of cells, degree of deformation of the cells (degree of juvenile of the cells) and kind of the cells are measured by performing the image processing and the result of the measurement is used as a parameter of the cell activation degree so that the administration of the bioactive substance such as lymphokine, e.g., interleukin 2 is controlled.

Another example of the culture monitoring unit 30 is explained with reference to Fig. 8.
(A) TARGET CELL INFUSION: Target cells (such as K-562 cell line) which show activity under a culture operation are infused in response to a transfer path signal, transfer mechanism signal, preliminary processing manipulator signal, pump signal, preliminary manipulator signal and pump signal. A measurement vessel 501 is transferred from a stock room 502 to a work station. The target cells are infused in the vessel 501 by a manipulator 504, pipet 503 and pump 506. Dyeing fluid is infused in the same vessel 501. The vessel is transferred into a CO₂ incubator 507. In the operation mentioned above, (1) the cells in the dyeing fluid may be previously infused. (2) The dyed cells may be used in the operations below (C) to be mentioned. The purpose of the work is that dyeing substance (such as ⁵¹Cr or Eu) is taken into the target cells.
(B) CLEANING: The vessel is taken out from the CO₂ incubator 507 and is transferred to the work station in response to a signal sent from the controller, transfer mechanism signal, transfer path signal, preliminary processing manipulator signal and pump signal after a predetermined period. The target cell suspension in the vessel is infused in a centrifugal tube 510 through the preliminary processing manipulator 504, pipet 503 and pump 506. The target cell suspension is centrifuged in response to a centrifugal device signal and a centrifugal unit 511 is activated in order to collect the cell differentiation and is stopped after a predetermined time (5 to 10 minutes). Sensing of the cell differentiation is performed by the sensor 40. The sensing signal is used as a differentiation position data. The supernatant fluid is excluded by the pipet 503, manipulator 504 and pump 506 with reference to the sensing data in response to the preliminary processing manipulator signal and pump signal. The culture medium is infused in the centrifugal tube 510 in response to the preliminary processing signal and pump signal. The pipet 503 is inserted into the centrifugal tube and the pump 506 is operated to suck and discharge in response to a preliminary processing manipulator signal and pump signal, whereby the solution is stirred.
(C) INFUSION OF CULTURE CELLS: The target cell suspension is transferred from the centrifugal tube to a measurement vessel 2 and the cells cultured in the culture tank are sampled and infused in the same vessel 2. The measurement vessel 2 is transferred from the stock room to the work station in response to the transfer path signal and carrying out mechanism signal. The pipet 503, pump 506 and preliminary processing manipulator 504 are driven in response to the pump signal and preliminary processing manipulator signal so that the target cell suspension is infused from the centrifugal tube 510 into the measurement vessel 2. The culture cell suspension transferred from the culture pipe 17 in the culture tank 14 is infused through the pump 508 and the cell suspension infusion manipulator 505 in response to the pump signal. The measurement vessel 2 is stored in the CO₂ incubator 507 by using the transfer path 512 in response to the transfer path signal and carrying in/out mechanism signal.
(D) PRELIMINARY PROCESS FOR MEASUREMENT: The measurement vessel 2 is carried out of the CO₂ incubator 507 and transferred to the work station by using the transfer path 512 and carrying in/out mechanism in response to the carrying in/out mechanism signal and the transfer path signal. The mixed cell suspension is transferred to the centrifugal tube 510 by using the pump 506, preliminary processing manipulators 503 and 504 in response to the pump signal and manipulator signal. The mixed cell suspension is centrifuged in response to the centrifugal device signal so that the cell differentiation (5 to 10 minutes) is separated. The cell differentiation is sensed by the sensor 25 so that the sensing signal differentiated position data are sent. A measurement vessel 3 is carried out of the stock room 502 and transferred to the work station in response to the carrying in/out mechanism signal and transfer path signal. The supernatant of the mixed cell suspension is taken (based on the differentiation position data) and infused into the measurement vessel 3 by using the pipet 503, pump 506 and preliminary processing manipulator 504 in response to the pump signal and preliminary processing manipulator signal. By the operation mentioned above, the measurement vessel 2 is carried out and the mixed cell suspension is transferred to the centrifugal tube so as to be centrifuged after a predetermined period in response to the signal sent from the controller.
(E) MEASUREMENT: The measurement vessel 3 is transferred to a measurement room 509 in response to the transfer path signal so as to be measured. The output signal of the measurement room is transmitted to the controller 50. In case fluorescent substance such as Eu (europium) is used in the dyeing fluid, fluorescent measurement is performed. In case of using radioactive isotope such as ⁵¹Cr, RI measuring instrument is used.

In addition, the principle of the measurement is that Eu is taken into the target cells during the culture. The Eu is discharged outside the target cell due to the killer activity of the cell undergoing the culture.

Moreover, in case of using ⁵¹Cr(RI) instead of Eu, the principle is same as above.

Data for judging whether or not the cells in the culture have killer activity are gathered. On the basis of the data, the administration of the bioactive substance such as lymphokine, e.g., interleukin 2 is controlled.

A series of measurements are performed under the control of the controller 50 at regular intervals.

The summary of the control of the culture described above is as follows.

### [1] CONTROL OF THE ADMINISTRATION OF THE BIOACTIVE SUBSTANCE

### (1) CONTROL BY THE NUMBER OF CELLS OR CELL DENSITY

The number of cells is measured by image processing performed by the culture monitoring unit and the cell density of the cells under culturing is calculated on the basis of the amount of the cell suspension measured by the controller. For example, in the case that the cell density measured under culturing is below the density set by the controller (example of set value: 5x10⁶cells/ml), e.g. interleukin 2 is added as a bioactive substance. The work mentioned above is done at the culture unit.

### (2) CONTROL BY CYTOTOXICITY

According to the measurement of the cytotoxicity by the culture monitoring unit, the cytotoxicity of the cells under cultivation is obtained. As a label of the cells, ⁵¹Cr, Eu and CFDA (carboxy fluo recceln Diacetate) can be used. When the cytotoxicity of the cells under cultivation is below the density set by the controller, for example, the obtained cytotoxicity is below 20% assuming that the rate is 100% when the cytotoxicity of all of the target cells is obtained, the bioactive substance e.g. interleukin 2 is added. The operating portion is the culture unit.

### (3) CONTROL BY KIND OF CELLS

The kind of the cells under culturing is measured by an image processing using a fluorescent image by means of the culture monitoring unit or by a light measurement device using a fluorescent measurement device so that the distribution and rate thereof are obtained from the calculation performed by the controller. As a dyeing substance at this time, monoclonal antibody labelled with fluorescent substance may be used. When the rate of a peculiar kind of cells in the cells under culturing (e.g., NK cells; natural killer cells) is below the value (rate) set by the controller, the bioactive substance is added. The operation mentioned above is done in the culture unit.

### (4) CONTROL BY SHAPE OF CELLS

The shape parameter of the dyed and undyed cells is measured by the image processing performed by the culture monitoring unit. As a shape parameter, (i) the size of the cell, (ii) the flatness of the cell, e.g., may be used. In the case that the distribution or the mean value of the shape parameters of the cells under culturing is smaller than the value set by the controller, the bioactive substance, e.g., interleukin 2 is added. The operation mentioned above is done in the culture unit.

### [2] CONTROL OF EXCHANGE OF CULTURE MEDIUM

### (1) CONTROL BY NUMBER OF CELLS OR CELL DENSITY

The number of the cells is measured by the image processing performed by the culture monitoring unit so that the density of the cells is calculated by the controller based on the amount of the cell suspension. As the method of control, (i) for example, when the density of the cells under cultivation is beyond the density set by the controller, it is so controlled that the culture medium is exchanged at regular intervals, e.g., every day. (ii) When the density of the cells under cultivation is beyond the density set by the controller, it is so controlled that the cell suspension is diluted to the initial density. The operation mentioned above is made in the culture unit.

### (2) CONTROL BY pH VALUE

The pH value of the cell suspension or medium in which the cells are cultured is measured by means of the light measurement device of the culture monitoring unit. When the measured pH value of the medium shows the acid degree beyond the value set by the controller, the culture medium is exchanged. The operation mentioned above is made in the culture unit.

The summary of the control of recovering the cells is described as follows.

### [1] CONTROL BY NUMBER OF CELLS OR CELL DENSITY

The number of the cells is measured by the image processing performed by the culture monitoring portion and the density of the cells under cultivation is calculated by the controller based on the measured amount of the cell suspension. For example, when the measured density of the cells under cultivation is beyond the density of the cells set by the controller (example of set value; 1x10⁷cells/ml), a signal is transmitted from the separator unit to the culture unit and the recovery operation is started. The operations mentioned above are done in the separator and culture units.

### [2] CONTROL BY CYTOTOXICITY

The cytotoxicity of the cells under cultivation is obtained by measuring the cytotoxicity with the culture monitoring unit. As a label of the cells, CFDA, ⁵¹Cr and Eu can be used. When the cytotoxicity of the cells under cultivation becomes larger than the value set in the controller (for example, assuming that the value is 100% when all of the target cells are toxic, in the case the obtained cytotoxicity is over 30%), the control signals are sent from the controller to the separator and culture units so that the recovery operation is started. The operation mentioned above is done in the separator and culture units.

### [3] CONTROL BY THE KIND OF CELLS

The kind of the cells, under cultivation is measured by the image process using fluorescent image performed by the culture monitoring unit or by the light measurement device using a fluorescent light measurement device so that the distribution of the rate thereof is obtained by the calculation performed by the controller. At this time, as a dyeing material, a monoclonal antibody labelled with fluorescent materials (e.g., FITC, P.E., RITC) may be used. When the rate of a specific kind of cells under cultivation (e.g., NK cell) is beyond the value (rate) set in the controller, the signals are sent from the controller to the separator and culture portions so that the recovery operation is started. The operation mentioned above is done in the separator and culture units.

### [4] CONTROL BY THE SHAPE OF CELLS

The shape parameters of the dyed and undyed cells are measured by the image process performed by the culture monitoring unit. As the shape parameters, (i) the size of the cells, (ii) flatness of the cells may be used for example. When the distribution or the mean value of the shape parameters of the cells under cultivation becomes larger than the value set in the controller, the control signals are sent from the controller to the separator unit and culture units so that the recovery operation is started. The operation is done in the culture unit. In each of the control operations, the combination of more than two controls mentioned above may be used and the present invention is not limited to the controls mentioned above.

The effects of the present invention are described as follows.
(1) The growth and activation of the cells can be easily performed by simply setting the condition of each of the mechanisms and starting the operations of the device without any specific technique, the labor, especially the labor of a medical doctor can be remarkably reduced and in the case of application for therapy, one doctor can care more patients than before.
(2) If the entire part of the device is set in a clean bench or safety cabinet, the operation can be performed under the aseptic condition without a help of human hand, therefore, the possibility of the contamination of various bacteria is low so that the device applied for therapy has a high safety. Moreover, also for the medical doctor, there is no fear of infection of pathogenic fungi.
(3) In the prior art of the cell growth method manually operated, since the cell density and the activity of the cells were not measured in many cases, the optimum condition for the growth and activity of the cells can not be obtained. In the device according to the present invention, since the density and the activity of the cells can be suitably measured, e.g., at the time of setting so that the culture condition can be dynamically controlled, therefore, the optimum cultivation can be performed, so that the density and activity of the cells can be much more heightened than in the prior art and the cells can be stably provided.
(4) Even in the case the difference of the cell characteristics due to the patients is included and many kinds of cells are cultured, there is few risks of taking a cultivation method by mistake, so that the cultivation can be performed in safety at the same time.

## Claims

1. A cell proliferation device comprising:
separator means (20) for separating cells;
culture means (10) having a culture tank (14) for cultivating the cells, transfer means (13) for transferring the cells between said separator means (20) and said culture means (10); and
controller means (50) for controlling the operations of the separator means (20) and the transfer means (13),
**characterized** in
that said separator means (20) comprise a centrifugal device (22) capable of centrifuging a solution, a pipetting device (24) capable of absorption and discharge of the solution, a manipulator (23) for manipulating said pipetting device (24), and a sensor (25) for sensing the position of each of cell differentiations after centrifugation, so that the pipetting operation is controlled on the basis of the sensing result of each of the differentiation positions,
that culture monitoring means (30) are provided for measuring the characteristics of the cells stored in said culture means (10), and
that said controller means (50) calculate the characteristics of the cells obtained by said culture monitoring means (30) and for controlling the culture condition of the culture tank (14), and for controlling the operations of said separator means (20), said transfer means (13) and said culture monitoring means (30).

2. The cell proliferation device according to claim 1, wherein the centrifugal operation of said separator unit (20) is a continuous centrifugation.

3. The cell proliferation device according to claim 2, wherein a density gradient centrifugation is used for separating the cells.

4. The cell proliferation device according to claim 1, wherein said separator unit (20) comprises a membrane filter (602) for separating the cells, a tool for supporting said membrane filter and a fluid path, so that the cells are separated by filtration.

5. The cell proliferation device according to claim 4, wherein said membrane filter (602), tool for supporting the membrane filter and fluid path can be reversely purified.

6. The cell proliferation device according to claim 1, wherein said separator unit (20) separates the cells using differences of quantity of electro charge on the cells.

7. The cell proliferation device according to claim 6, wherein said separator unit (20) comprises a tank having electrodes for applying voltage to the solution and a fluid path.

8. The cell proliferation device according to claim 1, wherein said culture tank (14) is capable of infusing and discharging a culture solution.

9. The cell proliferation device according to claim 1, wherein said culture tank (14) is detachably provided in the culture unit (10) and said transfer means (13) transfers said culture tank (14) between the culture unit (10) and the separator unit (20).

10. The cell proliferation device according to claim 1, wherein the operations of the separator unit (20), culture unit (10) and transfer means (13) can be performed under an aseptic condition, respectively.

11. The cell proliferation device according to claim 1, wherein said device furthermore comprises sampling means for sampling the cell suspension stored in the culture tank (14).

12. The cell proliferation device according to claim 11, wherein a culture monitoring unit (30) comprises dyeing means for dyeing the cells or label means for labelling the cells.

13. The cell proliferation device according to claim 12, wherein said culture monitoring unit comprises measuring means for measuring the characteristics of the cells.

14. The cell proliferation device according to claim 13, wherein the image processing of the images of the cells obtained through a microscope is performed by said measuring means.

15. The cell proliferation device according to claim 13, wherein said measuring means comprises a light measurement device.

16. The cell proliferation device according to claim 13, wherein said measuring means measures the quantity of radioisotope.

17. The cell proliferation device according to claim 1, wherein the culture condition is controlled by controlling the administration amount of bioactive substance using the measurement result of the characteristics of the cells.

18. The cell proliferation device according to claim 1, wherein the culture condition is controlled by controlling the exchange of the culture medium using the measurement result of the characteristics of the cells.

19. The cell proliferation device according to claim 1, wherein the timing of recovering the cells is controlled using the measurement result of the characteristics of the cells.

20. The cell proliferation device according to claim 19, wherein the characteristic of the cells to be measured is the number of the cells or the density of the cells.

21. The cell proliferation device according to claim 19, wherein the characteristic of the cells to be measured is a cytotoxicity.

22. The cell proliferation device according to claim 19, wherein the characteristic of the cells to be measured is the kind of the cells or the phenotype thereof.

23. The cell proliferation device according to claim 19, wherein the characteristic of the cells to be measured is the shape of the cells, size of the cells or deformation degree of the cells.

24. The cell proliferation device according to claim 1, wherein at least one of the pH value, temperature, humidity or CO₂ density of the medium is measured and the measured value is controlled in a feed-back manner.

## Patentansprüche

1. Zellproliferationsvorrichtung, umfassend:
eine Trenneinrichtung (20) zum Trennen von Zellen;
eine Kultureinrichtung (10) mit einem Kulturtank (14) zum Kultivieren der Zellen, eine Transfereinrichtung (13) zum Überführen der Zellen zwischen der besagten Trenneinrichtung (20) und der besagten Kultureinrichtung (10); und
eine Steuereinrichtung (50) zum Steuern des Betriebs der Trenneinrichtung (20) und der Transfereinrichtung (13),
dadurch gekennzeichnet,
daß die besagte Trenneinrichtung (20) eine zum Zentrifugieren einer Lösung geeignete Zentrifugeneinrichtung (22), eine zum Aufnehmen und Abgeben der Lösung geeignete Pipettiereinrichtung (24), einen Manipulator (23) zum Manipulieren der besagten Pipettiereinrichtung (24) und einen Sensor (25) zum Abtasten der Position von jeder von Zelldifferentiationen nach einem Zentrifugieren umfaßt, so daß der Pipettiervorgang auf der Grundlage des Abtastergebnisses von jeder der Differentiationspositionen gesteuert wird,
daß eine Kulturüberwachungseinrichtung (30) vorgesehen ist, um die Eigenschaften der in der besagten Kultureinrichtung (10) eingelagerten Zellen zu messen, und
daß die besagte Steuereinrichtung (50) die mittels der besagten Kulturüberwachungseinrichtung (30) erhaltenen Eigenschaften der Zelle berechnet, sowie zum Steuern der Kulturbedingungen des Kulturtanks (14) und zum Steuern des Betriebs der besagten Trenneinrichtung (20), der besagten Transfereinrichtung (13) und der besagten Kulturüberwachungseinrichtung (30).

2. Zellproliferationsvorrichtung nach Anspruch 1, dadurch gekennzeichnet, daß der Zentrifugiervorgang der besagten Trenneinheit (20) ein kontinuierliches Zentrifugieren ist.

3. Zellproliferationsvorrichtung nach Anspruch 2, dadurch gekennzeichnet, daß ein Dichtegradienten-Zentrifugieren zum Trennen der Zellen verwendet wird.

4. Zellproliferationsvorrichtung nach Anspruch 1, dadurch gekennzeichnet, daß die besagte Trenneinheit (20) einen Membranfilter (602) zum Trennen der Zellen, ein Gerät zum Halten des besagten Membranfilters und einen Flüssigkeitspfad umfaßt, so daß die Zellen durch Filtration getrennt werden.

5. Zellproliferationsvorrichtung nach Anspruch 4, dadurch gekennzeichnet, daß der besagte Membranfilter (602), das besagte Gerät zum Halten des Membranfilters und der Flüssigkeitspfad im Gegenstrom gereinigt werden können.

6. Zellproliferationsvorrichtung nach Anspruch 1, dadurch gekennzeichnet, daß die besagte Trenneinheit (20) die Zellen unter Verwendung von Unterschieden der Menge an elektrischer Ladung auf den Zellen trennt.

7. Zellproliferationsvorrichtung nach Anspruch 6, dadurch gekennzeichnet, daß die besagte Trenneinheit (20) einen Tank mit Elektroden zum Anlegen einer Spannung an die Lösung und einen Flüssigkeitspfad umfaßt.

8. Zellproliferationsvorrichtung nach Anspruch 1, dadurch gekennzeichnet, daß der besagte Kulturtank (14) zum Infundieren und Abgeben einer Kulturlösung geeignet ist.

9. Zellproliferationsvorrichtung nach Anspruch 1, dadurch gekennzeichnet, daß der besagte Kulturtank (14) abnehmbar in der Kultureinheit (10) vorgesehen ist, und daß die besagte Transfereinrichtung (13) den besagten Kulturtank (14) zwischen der Kultureinheit (10) und der Trenneinheit (20) überführt.

10. Zellproliferationsvorrichtung nach Anspruch 1, dadurch gekennzeichnet, daß der Betrieb der Trenneinheit (20), der Kultureinheit (10) und der Transfereinrichtung (13) jeweils unter aseptischen Bedingungen durchgeführt werden können.

11. Zellproliferationsvorrichtung nach Anspruch 1, dadurch gekennzeichnet, daß die besagte Vorrichtung außerdem eine Beprobungseinrichtung umfaßt, um die in den Kulturtank (14) eingelagerte Zellsuspension zu beproben.

12. Zellproliferationsvorrichtung nach Anspruch 11, dadurch gekennzeichnet, daß eine Kulturüberwachungseinheit (30) eine Färbeeinrichtung zum Färben der Zellen oder eine Markiereinrichtung zum Markieren der Zellen umfaßt.

13. Zellproliferationsvorrichtung nach Anspruch 12, dadurch gekennzeichnet, daß die besagte Kulturüberwachungseinheit eine Meßeinrichtung zum Messen der Eigenschaften der Zellen umfaßt.

14. Zellproliferationsvorrichtung nach Anspruch 13, dadurch gekennzeichnet, daß die Bildverarbeitung der durch ein Mikroskop erhaltenen Bilder der Zellen von der besagten Meßeinrichtung durchgeführt wird.

15. Zellproliferationsvorrichtung nach Anspruch 13, dadurch gekennzeichnet, daß die besagte Meßeinrichtung eine Lichtmeßvorrichtung umfaßt.

16. Zellproliferationsvorrichtung nach Anspruch 13, dadurch gekennzeichnet, daß die besagte Meßeinrichtung die Radioisotopmenge mißt.

17. Zellproliferationsvorrichtung nach Anspruch 1, dadurch gekennzeichnet, daß die Kulturbedingungen durch Steuerung der Verabreichungsmenge an bioaktiver Substanz unter Verwendung des Meßergebnisses der Eigenschaften der Zellen gesteuert werden.

18. Zellproliferationsvorrichtung nach Anspruch 1, dadurch gekennzeichnet, daß die Kulturbedingungen durch Steuerung des Austauschs des Kulturmediums unter Verwendung des Meßergebnisses der Eigenschaften der Zellen gesteuert werden.

19. Zellproliferationsvorrichtung nach Anspruch 1, dadurch gekennzeichnet, daß der Zeitpunkt einer Rückgewinnung der Zellen unter Verwendung des Meßergebnisses der Eigenschaften der Zellen gesteuert wird.

20. Zellproliferationsvorrichtung nach Anspruch 19, dadurch gekennzeichnet, daß die zu messende Eigenschaft der Zellen die Anzahl der Zellen oder die Zelldichte ist.

21. Zellproliferationsvorrichtung nach Anspruch 19, dadurch gekennzeichnet, daß die zu messende Eigenschaft der Zellen eine Cytotoxizität ist.

22. Zellproliferationsvorrichtung nach Anspruch 19, dadurch gekennzeichnet, daß die zu messende Eigenschaft der Zellen die Art der Zellen oder deren äußeres Erscheinungsbild ist.

23. Zellproliferationsvorrichtung nach Anspruch 19, dadurch gekennzeichnet, daß die zu messende Eigenschaft der Zellen die Form der Zellen, die Größe der Zellen oder der Grad der Deformation der Zellen ist.

24. Zellproliferationsvorrichtung nach Anspruch 1, dadurch gekennzeichnet, daß von pH-Wert, Temperatur, Feuchtigkeit oder CO₂-Dichte des Mediums mindestens einer gemessen wird, und der gemessene Wert in einer Art Rückkopplung geregelt wird.

## Revendications

1. Dispositif de prolifération de cellules comprenant :
un dispositif de séparation (20) pour séparer des cellules ;
un dispositif de culture (10) ayant un réservoir de culture (14) pour cultiver les cellules, un dispositif de transfert (13) pour transférer les cellules entre ledit dispositif séparateur (20) et ledit dispositif de culture (10); et
un dispositif de commande (50) pour commander le fonctionnement du dispositif séparateur (20) et du dispositif de transfert (13),
caractérisé en ce que
ledit dispositif de séparation (20) comprend un dispositif centrifugeur (22) capable de centrifuger une solution, un dispositif de pipette (24) capable d'absorber et de décharger de la solution, un manipulateur (23) pour manipuler ledit dispositif de pipette (24), et un détecteur (25) pour détecter la position de chacune des différenciations de cellules après centrifugation, de sorte que le fonctionnement de la pipette est commandée sur la base du résultat de détection de chacune des positions de différenciation,
en ce que les dispositifs de surveillance (30) de culture sont prévus pour mesurer les caractéristiques des cellules stockées dans ledit dispositif de culture (10), et
en ce que lesdits dispositifs de commande (50) calculent les caractéristiques des cellules obtenues par lesdits dispositifs de surveillance (30) de culture et pour commander les conditions de culture du réservoir (14) de culture, et pour commander les fonctionnements dudit dispositif séparateur (20), dudit dispositif de de transfert (13) et desdits dispositifs de surveillance de culture (30).

2. Dispositif de prolifération de cellules selon la revendication 1, dans lequel l'opération de centrifugation de ladite unité de séparation (20) est une centrifugation continue.

3. Dispositif de prolifération de cellules selon la revendication 2, dans lequel on utilise une centrifugation du gradient de densité pour séparer les cellules.

4. Dispositif de prolifération de cellules selon la revendication 1, dans lequel ladite unité de séparation (20) comprend un filtre à membrane (602) pour séparer les cellules, un outil pour supporter ledit filtre à membrane et un circuit de fluide, de sorte qu'on sépare les cellules par filtrage.

5. Dispositif de prolifération de cellules selon la revendication 4, dans lequel ledit filtre à membrane (602), l'outil pour supporter le filtre à membrane et le circuit de fluide peuvent être purifiés de façon réversible.

6. Dispositif de prolifération de cellules selon la revendication 1, dans lequel ladite unité de séparation (20) sépare les cellules en utilisant des différences de quantité de charge électrique sur les cellules.

7. Dispositif de prolifération de cellules selon la revendication 6, dans lequel ladite unité de séparation (20) comprend un réservoir ayant des électrodes pour appliquer une tension à la solution et à un circuit de fluide.

8. Dispositif de prolifération de cellules selon la revendication 1, dans lequel ledit réservoir de culture 14 est capable d'injecter et de décharger une solution de culture.

9. Dispositif de prolifération de cellules selon la revendication 1, dans lequel ledit réservoir de culture (14) est placé de façon détachable dans ladite unité de culture (10) et ledit dispositif de transfert (13) transfère ledit réservoir de culture (14) entre l'unité de culture (10) et l'unité de séparation (20).

10. Dispositif de prolifération de cellules selon la revendication 1, dans lequel on peut réaliser respectivement, le fonctionnement de l'unité de séparation (20), de l'unité de culture (10) et des dispositifs de transfert (13) dans conditions aseptiques.

11. Dispositif de prolifération de cellules selon la revendication 1, dans lequel ledit dispositif comprend de plus, des dispositifs d'échantillonnage pour échantillonner la suspension de cellules stockée dans le réservoir de culture (14).

12. Dispositif de prolifération de cellules selon la revendication 11, dans lequel une unité de surveillance de culture (30) comprend des dispositifs de teinture pour colorer les cellules ou des dispositifs de marquage pour marquer les cellules.

13. Dispositif de prolifération de cellules selon la revendication 12, dans lequel ladite unité de surveillance de la culture comprend des dispositifs de mesure pour mesurer les caractéristiques des cellules.

14. Dispositif de prolifération de cellules selon la revendication 13, dans lequel le traitement d'images des images des cellules obtenues via un microscope est réalisé par lesdits dispositifs de mesure.

15. Dispositif de prolifération des cellules selon la revendication 13, dans lequel lesdits dispositifs de mesure comprennent un dispositif de mesure de la lumière.

16. Dispositif de prolifération de cellules selon la revendication 13, dans lequel lesdits dispositifs de mesure mesurent la quantité de radio-isotopes.

17. Dispositif de prolifération de cellules selon la revendication 1, dans lequel les conditions de culture sont contrôlées par la régulation de la quantité administrée de substance bioactive en utilisant le résultat de la mesure des caractéristiques des cellules.

18. Dispositif de prolifération des cellules selon la revendication 1, dans lequel les conditions de culture sont contrôlées par la régulation de l'échange du milieu de culture en utilisant le résultat de la mesure des caractéristiques des cellules.

19. Dispositif de prolifération de cellules selon la revendication 1, dans lequel on commande le temps de récupération des cellules en utilisant le résultat de la mesure des caractéristiques des cellules.

20. Dispositif de prolifération de cellules selon la revendication 19, dans lequel la caractéristique des cellules à mesurer est le nombre de cellules ou la densité des cellules.

21. Dispositif de prolifération de cellules selon la revendication 19, dans lequel la caractéristique des cellules à mesurer est une cytotoxicité.

22. Dispositif de prolifération de cellules selon la revendication 19, dans lequel la caractéristique des cellules à mesurer est le type des cellules ou le phénotype de celles-ci.

23. Dispositif de prolifération de cellules selon la revendication 19, dans lequel la caractéristique des cellules à mesurer est la forme des cellules, la dimension des cellules ou le degré de déformation des cellules.

24. Dispositif de prolifération de cellules selon la revendication 1, dans lequel on mesure au moins une des valeurs de pH, de température, d'humidité ou de densité de CO₂ du milieu et la valeur mesurée est commandée d'une manière rétroactive.
